# EUROPEAN PATENT APPLICATION

(11) **EP 2 107 126 A1**
(43) Date of publication of application: **07.10.2009**
(21) Application number: 08153856.3
(22) Date of filing: 31.03.2008
(51) Int. Cl.: C12Q 1/68

(54) **Detection of unspecified genetically modified organism (GMO) on micro-arrays**

(71) Applicant: Eppendorf Array Technologies SA, 5000 Namur (BE)
(72) Inventor: Remacle, José, B-5020, Malonne (BE); Hamels, Sandrine, B-6280, Joncret (BE)
(74) Representative: pronovem

(57) **Abstract**

The present invention is related to a method, kit and computer program for detecting the presence in a sample of an unspecified Genetically Modified Organism (GMO).

## Description

### Field of the invention

The present invention is in the field of diagnosis and is related to a method and kit comprising reagents and means for a detection of unspecified genetically modified organisms (GMO) such as plants, animals, bacteria and/or yeasts that may be present in a sample.

The invention is especially suited to discriminate between the presence of authorized and unauthorized GMOs in food control in countries where the regulation authorizes the presence upon their market of only a limited number of some specified GMOs.

### Background of the invention

The development of the biochips technology allows a simultaneous detection of multiple and specific nucleotide sequences present in a given sample and thus allows the identification of the corresponding organism or parts of the organism from which these sequences are obtained. Micro-arrays are solid supports containing on their surface a series of discrete regions bearing capture polynucleotide sequences (or capture probes) that are able to bind (by hybridisation) to a corresponding target nucleotide sequence(s) possibly present in a sample to be analysed. Their detections have to be worked out in conjunction with their genetic amplification, such as PCR.

The present invention makes use of the possibilities offered by multi-parametric assay for the detection of unspecified GMOs.

### State of the art

Micro-arrays are well suited for multi-parametric detections both for protein and for polynucleotide sequences. The material to be analyzed is usually in the sample in very low amount and a detection of a genetic material needs first to be amplified. Among the genetic amplification methods, the PCR is the most common one. The difficulty for the amplified sequences (amplicons) to be detected on immobilized capture molecules is the fact that they are double stranded and thus reassociate very fast in solution so that it is difficult to get a signal for their identification and possibly their quantification. This issue was resolved and is presented in the patent US 7,205,104 and US 7,202,026. Other strategies exist, which are based on the use of single stranded sequences or on the fragmentation of the amplified sequences see WO 97/29212 and WO98/28444.
The detection of GMOs needs a combination between PCR amplification and micro-array detection techniques.

GMOs contain by definition a transformation event that usually means insertion of a nucleic acid construct or cassette into a recipient organism. This gene construct is composed of several genetic elements, usually at least one gene of interest, a promoter functioning as start signal, and a terminator functioning as a stop signal for regulation of gene expression. In addition, the construct may be flanked by DNA sequences from the cloning vector. The majority of genetically modified (GM) plants have been transformed with constructs containing the Cauliflower Mosaic Virus (CaMV) 35S promoter (P-35S) and/or the CaMV 35S terminator (T-35S) and/or the *Agrobacterium tumefaciens* nopaline synthase terminator (T-Nos). The most commonly used cloning vectors, are pBR322 containing a gene coding for resistance to ampicillin (bla) antibiotics, or vectors containing a gene coding for resistance to neomycin/kanamycin (*nptII*) antibiotics.

Several countries have mandatory labelling regulations for GMOS and for GM-derived food compositions. Particularly, European Union regulations establish the compulsory labelling of food and feed consisting of or containing genetically modified organisms (GMOs) in a proportion higher than 0.9 percent of the food ingredients (Regulation EC no. 1829/2003 and 1830/2003) for the traceability of the GMOs in food. The list of the authorized GMO in the EU is published on the web site http://ec.europa.eu/food/dyna/gm_register/index_en.cfm and applications for new GMO have been introduced to the EU regulatory administration for approval (http://gmo-crl.jrc.it/statusofdoss.htm. The number of GMOs concerned by this regulation changes with time: there were 12 GMOS approved in 1999, 20 in 2006 and 24 in 2007. The GMOs which are not in this list are considered as non-approved. The EU regulation requires for a GMO to be approved that a method of testing is provided by the company. So when a GMO is accepted information is publicly available for a specific testing of the approved GMOS. For the non-approved GMOs, the information is usually not available and this is the reason why they are sometimes listed as unknown GMOs (or unspecified GMOs).

Other countries such as Japan, Taiwan and South Korea have established a 5% threshold. South American countries as Brazil adopted a threshold to label all products containing more than 4% of GMO. Some countries like Australia and New Zealand also require labelling of the food for the GMO.

In order to comply with the national and European requests of those regulations and to guarantee to the consumers a freedom of choice by accurate information, several GMO analytical methods have been already described and have been validated by the CRL Community Reference laboratory (CRL) in collaboration with the European Network of GMO Laboratories or ENGL (http://gmocrl.jrc.it/statusofdoss.htm).

GMO testing may be limited to a simple screening PCR which is commonly done on some genetic elements commonly found in the GMO like the P35S or T-Nos. However, specific identifications and quantifications are needed to comply with the requirement of the regulation and the labelling. Individual test of each GMO becomes difficult and costly in particular as the number of GMO to detect is continually increasing.

Because of the sensitivity to be reached, genetic material from the sample is first amplified by PCR before detection. PCR-based GMO tests can be grouped into categories corresponding to their level of specificity. Each category corresponds to the composition of the DNA fragment that is amplified in the PCR: screening targets, gene specific targets, construct specific or event-specific targets.

The first category of PCR methods, targeting the P-35S, T-35S, T-Nos, bla or nptII genetic elements, have wide applications for screening for genetically modified material (Matsuoka et al., 2002, J. Agric. Food Chem. 93: 35-38). However, these screening methods cannot be used to identify the GMO, since the presence of one of the screening targets does not necessary imply the presence of GMO-derived DNA. The source of P-35S or T-35S sequences could be naturally occurring CaMV sequences (Wolf et al. 2000, Eur. Food Res Technol. 210:367-372).

The second category of genetic amplification (PCR) methods, targeting a gene of interest (e.g. CryIA(b) gene), are more specific than the screening methods (Vaitilingom et al. 1999, J. Agric. Food Chem. 47: 5261-5266). The choice of available genes is greater than the choice of available promoter and terminator sequences. Normally a positive signal for the amplification of a specific gene implies that GM-derived DNA is present, and in many cases it is possible to identify from which GMO the DNA is derived.

The third category of PCR methods targets event-specific elements such as junctions between adjacent genetic elements of the gene construct (specific of the construct), for example a junction between a promoter sequence and a gene of interest (e.g. Mon810 maize: P-35S-hsp70 intron I) (Zimmerman et al. 1998, Lebensm-Wiss u Technol. 31: 664-667). With these methods a positive signal will only appear in the presence of GM-derived material. However, the full gene construct may have been transformed into more than one GMO, or may be used in future transformation.

The only unique signature of a transformation event, within the limitations of present day technology, is the junction at the integration locus between the recipient genome and the inserted DNA also called the edge fragment. This junction can be the target of the fourth category of PCR methods. Unfortunately, even the event-specific detection methods have their limitations. When two GMOs are crossed (e.g. two different GM maize such as T25 and Mon810), the resulting hybrid offspring usually contains the genetic modifications including signatures of both events and will be undistinguishable from its two parents in a PCR test. One limitation of this detection is the requirement of one specific primer pair for each GMO to identify.

Some of these assays are based on conventional PCR and allow a qualitative approach of the GMO in a given sample. Other such as the real-time PCR (Hernández et al., 2003, Transgenic Research 12: 179-189) allow the quantification of GMO in a sample.

The major limitations for (PCR-based) detection of DNA sequences derived from GMOs are access to information about applicable PCR primers and access to DNA sequence suitable for reliable analysis. The most complete data bases are the http://www.agbios.com and http://www.bats.ch/gmo-watch/ which collect the data on the common genetic elements found in the insert of the different GMOs. However, a sequence along edge fragments is difficult to obtain for some GMOs. Fortunately, the actual European legislation requests the companies to provide a specific assay for new GMOs. Such assay is usually a real time PCR on edge fragments so that primers and capture probe sequences are available for such authorized GMOs.

Because of the increasing number of GMOs which are accepted for their use in the EU, there is a need for multiplex assays for these accepted GMOs. In an attempt to perform a multiparametric assay, the US-20030198943A1 described a method for the identification of PCR-amplified sequences of GMOs on biochip. The PCR amplification is based on the use of a consensus primer pair (P-35S/T-35S) which delimits very long DNA sequences, including several genetic elements (e.g. CTP1, CTP2, CTP4, CP4EPSPS, EPSPS, CryIA(b), hsp70 intron, Pat) found in different GMOs. The capture molecule corresponding to one amplified sequence (amplicon) is selected in a genetic element (e.g. CTP1, CTP2, EPSPS, Cry1ab, hsp70 Int, Pat) delimitated by the consensus primers. Amplification of long fragments makes the method restricted to fresh leaves or seeds and not to processed food compositions where DNA sequences are fragmented. The method also presents rather limited flexibility for detection of additional GMOs.

A new method based on the analysis of a pattern of genetic elements present in the GMO insert together with the plant species was later proposed, which allows the identification of the known and accepted GMOs (EP1724360A1). This method indicates the presence of GMOs for which the pattern is known, but does not exclude the fact that other GMOs have the same pattern either identical or partially covered by this first pattern. Another restriction of this method is that the promoter (P-35S) commonly used in GMO is from Cauliflower Mosaic Virus (CaMV) and the terminator (T-Nos) is from *Agrobacterium tumefaciens* nopaline synthase, which can be present in fresh vegetables or in the environment in which they are grown.

GMO encompasses also organisms not incorporated in the data base(s) of the accepted GMOs for a particular country, but also a GMO for which information about its insert sequence is missing or a GMO for which a specific detection method is not available.

Facing a sample of unknown composition, it is impossible to perform a direct assay since no method that can detect a non-specified GMO is available.

### Aims of the invention

The present invention aims to solve a very peculiar problem, which is to detect a GMO plant, either unknown of the tester or unspecified or for which information is missing for its assay. Unexpectedly, the assay provides information regarding the presence of an unspecified GMO preferably without performing a specific assay targeting (only) such GMO. The present invention aims to provide a new method and tools (a kit or a device) to detect in a sample the presence of a Genetically Modified Plant (GM plant) or Genetically Modified Organism (GMO) which is not itemized as such in a predefined (known and possibly authorized in a specific country) list of GMOs (specified GMOs).

Another aim of the invention is to detect a GMO which in unauthorized by the legal authorities.

### Definitions

The terms "nucleic acid, oligonucleotide, array, micro-array, probe, target nucleic acid, bind substantially, hybridising specifically to, background, quantifying" are the ones described in the international patent application WO97/27317 incorporated herein by reference.

The terms "nucleotide triphosphate, nucleotide, primer sequence" are those described in the document WO00/72018 and PCT/BE00/00123 incorporated herein by references.

The terms "homologous genetic sequences" or "highly identical" mean amino acid or nucleotide sequences having a percentage of amino acids or nucleotides identical at corresponding positions which is higher than in purely random alignments. They are considered as homologous when they show a minimum of homology (or sequence identity) defined as the percentage of identical nucleotides or amino acids found at each position compared to a total of nucleotides or amino acids, after the sequences have been optimally aligned taking into account additions or deletions (like gaps) in one of the two sequences to be compared. Genes coding for a given protein but present in genetically different sources like different organisms, are usually homologous. Also in a given organism, genes coding for proteins or enzymes of the same family (Interleukins, cytochrome b, p. 450) are usually homologous. The degree of homology (or sequence identity) can vary a lot as homologous sequences may be homologous only in one part, a few parts or portions or all along their sequences. The parts or portions of the sequences that are identical in the sequences are perfectly homologous and are conserved or identical. Genetic construction as for cell transfection may contain identical or homologous parts such as for the terminators, promoters or the genes of interest. Protein domains which present a conserved three dimensional structure are usually coded by homologous sequences and even often by a unique exon. The sequences showing a high degree of invariance in their sequences are said to be highly conserved and they present a high degree of homology.

The terms "genetically modified organism" (GMO) or "GM event", correspond to all types of organisms or micro-organisms (including plants) which have been submitted to some genetic modifications, especially an insertion of a nucleic acid construct or cassette sequence comprising exogenous genetic elements to the recipient organism or micro-organism (including a plant): promoters and terminators sequences flanking a gene of interest and/or a genetic marker.

The term "genetic elements" refers to the presence of some specific nucleotide sequences which have a role or a function in the organism such as but not limited to a gene (hereafter referred to as gene specific sequences), a regulatory sequence, a promoter, a terminator or a repeat sequence (the three later being among screening sequences). These genetic elements comprise or correspond to nucleotide sequences, which can be common to many different GMOs (hereafter referred to as "common element"), which are specific of the plant species (referred to as "plant element") and which are sequences specific of a given GM event (GMO) the so called "event-specific elements".

The term "genetic map" is the organisation (presenting a specific pattern) of these various genetic elements in the genome of the organism, preferably in the genome of the plant.

The term "unspecified GMO" is a GMO which is not specified in a given list of specified GMO. The unspecified GMO are usually GMO for which a specific detection method is not available and/or which the specific edge fragment sequence is not used for the detection. It usually includes unauthorized GMOs for a given country (including countries of European Union). Unspecified GMO is often recalled as unknown GMO meaning a GMO which is not officially known or accepted in a specific country or a group of countries (European Union).

An "edge fragment" covers the junction sequence between the insert (cassette sequence comprising exogenous genetic elements to the plant) and the plant genome and is especially related to "event-specific element".

### Summary of the invention

The present invention relates to a method for detecting the presence in a sample of an unspecified Genetically Modified Organism (GMO) (which is preferably not part of a list of specified GMO), this method comprising the steps of:
(1) possibly extracting from the sample genetics elements comprising at least a portion of an exogenous nucleotide sequence integrated into the genome of the GMO,
(2) detecting the presence or the absence in the sample of one or more common genetic element nucleotide sequence(s) comprising screening and/or gene specific sequences being part of the said portion integrated into the genome of the GMO,
(3) detecting the presence or absence in the sample of one or more event-specific genetic element nucleotide sequence(s),
(4) identifying the presence or the absence of all specified GMO(s) present in the sample from the detection step (2) and/or (3),
(5) associating each common genetic element detected in step (2) to the said specified GMO(s) present in the sample and identified in step (4),
(6) deducing the presence of an unspecified GMO in the sample, when at least one of the common genetic element detected in step (2) is not associated with any of the specified GMO identified in the sample.

These screening and/or gene specific sequences include at least promoter, terminator, regulatory and/or repeat sequences, and possibly markers sequences, selection markers, genes of interest sequences, etc.

According to one aspect of the invention, event-specific genetic element is an edge fragment of a sequence present in the genome a specific GMO.

Preferably, the event-specific genetic element (nucleotide sequence) comprises at least 5 nucleotides on both sides of the edge fragment.

Advantageously, the method according to the invention contains an event-specific element for each of the specified GMO.

According to the invention, unspecified GMO is detected in the sample when at least one detected common genetic element is not present in the genetic map recalling the presence of the common genetic elements of the specified GMO.

In the method according to the invention, the detection of the presence or absence of the event-specific (step 3) and common genetic element nucleotide sequence(s) (step 2) is performed preferably in the same assay (simultaneously), possibly upon the same micro-array.

Preferably, these genetic elements are further quantified during the detection and a GMO abundance is deduced.

In the method of the invention, the genetic elements or part of them are preferably detected after amplification by PCR.

Preferably the primer for the common genetic elements are consensus primers for the (PCR) amplification of elements being homologous in at least 2 different specified GMO.

Specifically, the identification of the specified GMO(s) in the sample comprises a PCR amplification of the genetic elements nucleotide sequences and a detection of the amplified genetic elements on a micro-array.

Advantageously, the amplification of the genetic element nucleotide sequence(s) is performed by PCR genetic amplification using tailed primers and using second primers identical or complementary to the said tail(s). Preferably, these tailed primers and the second tail primer(s) are both present from the first amplification step of the PCR and the tailed primers are present (in the amplification step) in a concentration at least 5 times lower than the said tail primer(s).

Advantageously, the method according to the invention, further comprises a detecting step of the presence or absence (and possibly a quantifying step of the abundance) of one or more organism element nucleotide sequence(s) specific of the organism species, preferably specific of the plant species.

Preferably, these plant elements (plant species) are selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice) nucleotide sequences.

In the method according to the invention, the identification of the specified GMO(s) in the sample comprises a PCR amplification of the genetic element nucleotide sequence(s) and a detection of these amplified genetic element nucleotide sequence(s) on a micro-array.

Advantageously, in the method according to the invention, the event-specific genetic element is an edge fragment and event-specific genetic element nucleotide sequence is a nucleotide sequence comprising this edge fragment, preferably made of at least 10, 15, 20, 25, 30, 35 40, 45 or 50 nucleotides comprising at least 5, 7, 10, 15, 20 or 25 nucleotides on both sides of the insertion point (edge fragment).

In the method according to the invention the assay (means) contains event-specific element for at least 60%, preferably 80%, more preferably 100% of the specified GMO(s).

In the method according to the invention, the identification of the specified GMO(s) in the sample further comprises a real time PCR amplification of the event-specific genetic elements.

Advantageously, the common genetic element nucleotide sequence(s) are selected from the group consisting of P35S, T-Nos, Pnos-nptII, bla, nptII, Pat, Bar, Cry1Ab, Cry3Bb1 and EPSPS nucleotide sequence(s) being part of the screening and/or the gene specific elements.

Preferably, the specified GMO(s) are selected from the group comprising BT176, Mon810, Bt11, Mon863, GA21, Mon603, RRS, GT73, T45, Mon1445, Mon531, Mon15985, Mon863 X Mon810, Mon863 X Mon603, Mon603 X Mon810, GA21 X Mon810, Mon531 X Mon1445, Mon15985 X Mon1445, TC1507, Topas 19/2, H7-1, T25, DAS59122, TC1507 X Mon603, MS8 X RF3, MS1 X RF1, MS1 X RF2.

Preferably, the list of specified GMO comprises at least 5 and better 10 and even better 20 GMO from the list above.

Preferably, the list of specified GMO comprises RRS, MON810, Bt11, MON531 and T25 more preferably RRS and MON810 (75% of OGMs cultivated in 2008 in the world) or MON810 and T25 or Mon810, RRS and T25 (approved in the European Union).

Preferably, the list of specified GMO comprises at least 8 or all the (specified) GMOs selected from the group consisting of BT176, Mon810, Bt11, Mon863, GA21, Mon603, RRS, GT73, T45, Mon1445, Mon531, Mon15985, Mon863 X Mon810, Mon863 X Mon603, Mon603 X Mon810, GA21 X Mon810, Mon531 X Mon1445, Mon15985 X Mon1445, TC1507, Topas 19/2, H7-1, T25, DAS59122, TC1507 X Mon603, MS8 X RF3, MS1 X RF1, MS1 X RF2, and at least RRS, MON810, Bt11, MON531, MON15985, MON1445, T25 and Bt176.

The specified GMO(s) also preferably correspond to all approved GMO(s) in the European Union market.

Advantageously, the step(s) of detecting the presence or absence of one or more event-specific or common genetic element nucleotide sequence(s) is obtained by:
a) possibly extracting genetic elements corresponding to at least a portion of exogenous nucleotide sequence integrated into the genome of a GMO, from the sample;
b) amplifying or copying the said genetic element (or part(s) of it) nucleotide sequence(s) into target nucleotide sequences, having preferably a length comprised between (about) 50 and (about) 200 nucleotides;
c) possibly labelling the (possibly amplified) target nucleotide sequences;
d) putting into contact the target nucleotide sequences with capture polynucleotide sequences being specific of the amplified event specific genetic element(s) and being bound to an insoluble solid support, preferably a non porous solid support, containing at least 4 different bound capture polynucleotide sequences on different locations of solid support;
e) quantifying and/or recording for a target nucleotide sequence a signal at a specific location, said signal resulting from hybridization by complementary base pairing between the target nucleotide sequence and its corresponding capture polynucleotide sequence,
wherein the presence or absence of a signal on a capture polynucleotide sequence allows a detection and an identification (and/or a quantification) of the genetic element present in the sample.

Preferably, the step of identification of the specified GMO(s) in the sample comprises:
a) amplifying or copying a common genetic element or part of it into target nucleotide sequences having a length comprised between (about) 50 and (about) 200 bases; by using primers which are able to amplify the said common genetic element being homologous with the common genetic element present in at least two different specified GMOs;
b) possibly labelling the obtained target nucleotide sequences;
c) putting into contact the obtained target nucleotide sequences with single stranded capture polynucleotide sequences bound by a covalent link to an insoluble solid support at a specific location of a solid support surface;
d) detecting the binding of said (possibly labelled) target nucleotide sequences, by detecting a signal resulting from hybridization by complementary base pairing of said target nucleotide sequence and its corresponding capture polynucleotide sequence at the specific location, wherein the capture polynucleotide sequence(s) are bound to the insoluble solid support at a specific location according to a micro-array, having a density of at least 4, 20, 100, 400, 1000, 10.000 or more different bound single stranded capture nucleotide sequence(s)/cm² of solid support surface, wherein the capture polynucleotide sequence comprises a sequence having between (about) 10 and (about) 200 bases, which allows a specific hybridization with the target nucleotide sequence to be detected and/or quantified;
e) repeating the steps a) to d) for a second, third, fourth or more different common genetic elements specific of the specified GMO, and
f) identifying the specified GMO through the presence and absence of these different and multiple common genetic elements in the sample in comparison with their presence and absence in the GMO integrated exogenous nucleotide sequence (possibly presented according to a specific pattern in a database).

Preferably, the capture polynucleotide sequence for detecting the presence or absence of the event-specific and common genetic element of the GMO contain a sequence specific of the genetic elements of at least 15 and preferably at least 20 bases, but may comprises a sequence having a length between (about) 10 and (about) 200 nucleotides.

Advantageously, the capture polynucleotide sequence able to hybridise with a corresponding target nucleotide sequence is between (about) 10 and (about) 49 nucleotides in length and is separated from the surface of the solid support by a spacer of at least 6.8 nm being preferably a polynucleotide sequence of 20 and better at least 40 nucleotide long.

In the method of the invention, the binding between the target nucleotide sequence and its corresponding capture polynucleotide sequence preferably forms a single spot signal at a specific location.

Preferably, the spacer is a sequence of between (about) 20 and (about) 150 nucleotides.

According to the invention, the density of these capture nucleotide sequence bound to the surface at a specific location is preferably superior to 10 fmoles and preferably 100 fmoles per cm² of solid support surface.

According to one aspect of the invention, the amplified targets are copied into RNA sequences and/or cut into small polynucleotide sequences which are then hybridized on a plurality of capture molecules and the identity of the amplified targets is deduced from positive results on a plurality of capture molecules.

Preferably, the insoluble solid support is selected from the group consisting of glasses, electronic devices, silicon supports, plastic supports, compact discs, filters, gel layers, microbeads, metallic supports or a mixture thereof.

Another aspect of the invention is a micro-array and a diagnostic and/or quantification kit comprising the micro-array for detecting the presence in a sample of an unspecified Genetically Modified Organism (GMO), said micro-array comprising an insoluble solid support upon which at least 10, 20, 50 or more different (single stranded) capture polynucleotide sequences are bound at a density of at least 4, 20, 100, 1000, 4000, 10.000 or more single stranded capture polynucleotide sequences/cm² of solid support surface, said (single stranded) capture polynucleotide sequences being part of two families, the first one containing a sequence of between (about) 10 and (about) 60 bases, preferably of (about) 20 and (about) 40 bases, and having at least 10 consecutive nucleotides comprising the 5 nucleotides located on both sides of the insertion point of an event-specific genetic element nucleotide sequence and the second family having a sequence specific of GMO common genetic element nucleotide sequence(s), said solid support having at least 10, 20, 50 or more different capture polynucleotide sequences.

Advantageously in micro-array or kit according to the invention, the event-specific genetic element is an edge fragment.

The common genetic elements of the micro-array of the invention are selected from the group consisting of: P35s, T-nos, Pnos-nptII, bla, nptII, Pat, Bar, Cry1Ab, Cry3Bb1 and EPSPS.

In the micro-array or kit according to the invention, the capture probes specific of the event specific genetic element nucleotide sequences comprised at least 5 and better 10 and even better 20 among the BT176, Mon810, Bt11, Mon863, GA21, Mon603, RRS, GT73, T45, Mon1445, Mon531, Mon15985, TC1507, Topas 19/2, H7-1, T25, DAS59122, MS8, RF3, MS1, RF1, RF2 sequences, or preferably the others sequences above described.

The kit according to the invention, may further comprises primer pairs for (PCR) amplification of the event-specific genetic element and the common genetic element nucleotide sequence(s) of the GMO to be detected as targets on the capture polynucleotide sequences immobilized on the solid support.

In the Micro-array or kit according to the invention, the solid support may further comprise capture polynucleotide sequences for a specific detection of organism (preferably plant) elements, which are specific of the organism (plant) species, and specific primers pairs for (PCR) amplification of organism (plant) elements nucleotide sequence(s), which are specific of the organism (plant) species.

In the micro-array or kit according to the invention, these plant elements specific of plant species, are selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice).

In the kit according to the invention, the insoluble solid support comprises capture polynucleotide sequences for the detection of:
- at least 10 event-specific genetic element nucleotide sequence(s) of specified GMOs selected from the group consisting of : BT176, Mon810, Bt11, Mon863, GA21, Mon603, RRS, GT73, T45, Mon1445, Mon531, Mon15985, TC1507, Topas 19/2, H7-1, T25, DAS59122, MS8, RF3, MS1, RF1, RF2 sequences, and
- at least 3 common genetic element nucleotide sequence(s) selected from the group consisting of P35s, T-nos, Pnos-nptII, bla, nptII, Pat, Bar, Cry1Ab, Cry3Bb1 and EPSPS sequences.

In the micro-array and kit according to the invention, the insoluble solid support is selected from the group consisting of glasses, electronic devices, silicon supports, plastic supports, compact discs, gel layers, microbeads, metallic supports or a mixture thereof.

The kit according to the invention, may further comprises means in the form of a computer program for performing the identification of specified GMO in a sample and deducing the possible presence of an unspecified GMO in the sample, preferably from the different signals obtained on the capture polynucleotide sequences.

The computer program comprises a database collecting the genetic map of the different genetic element nucleotide sequence(s) of specified GMOs.

A last aspect of the invention is related to the computer program for detecting the presence in a sample of an unspecified Genetically Modified Organism (GMO) performing the steps of:
(1) listing specified GMO detected in the sample based on their corresponding event-specific genetic element nucleotide sequence(s) and/or on their corresponding pattern of common genetic element nucleotide sequence(s)possibly present in a database,
(2) determining a set of common genetic element(s) corresponding to the specified GMO listed in step 1,
(3) providing a list of common genetic element(s) detected in the sample,
(4) comparing the list obtained in step 3 to the set obtained in step 2 and deducing the presence of an unspecified GMO if at least one common genetic element detected in the sample in step 3 is not included in the set obtained in step 2.

### Brief description of the drawings

Figure 1 presents the outcome of the analysis of an unspecified GMO from list A as provided in table 1. The data analysis shows the presence of one specified GMO, MON810 and the presence of a common genetic element, the cry3b-1, as being part of an unspecified GMO.
Figure 2 presents the genetic elements detected for the specified GMOs defined in table 1.
Figure 3 presents the genetic elements detected for the specified GMOs defined in table 2.
Figure 4 presents the list of some unspecified GMOs for which information is provided in data bases (as compared to the specified GMOs listed in table 2). The figure also indicates some common genetic elements present in these unspecified GMOs.
Figure 5 represents the design of a micro-array for the detection of common genetic element and event-specific genetic element nucleotide sequences of the specified GMOs of list B as provided in table 2 and also presented in figure 4.
Figure 6 gives the results of the analysis of a food sample containing two specified GMOs. The protocol is given in example 1. Figure 6.A gives the picture of the micro-array analysis. Figure 6.B gives the analysis outcome as proposed in this invention. There was no unspecified GMO detected in the sample as compared to the specified GMOs of list B (table 2).
Figure 7 presents the outcome of the analysis of an unspecified GMO from list B as provided in table 2.

### Detailed description of the invention

The inventors have discovered that it is possible to solve a very peculiar problem which is to detect a GMO plant which is either unspecified or unknown of the tester and without having available an assay specific of this GMO.

The present invention discloses a method for detecting the presence in a sample of an unspecified Genetically Modified Organism (GMO) compared to a list of specified GMOs by the use of an assay of at least two different types of genetic elements, some being common to several GMOs and some being specific of a specified GMO.

This method makes use of the potentiality of the multiple data that can be obtained in one assay by the use of a multiplex PCR amplification followed by the detection of the various amplicons.

The method according to the invention surprisingly combines the identification of some common sequences of GMO referred as common genetic elements with the presence of sequences specific (event-specific element) of one GMO.

The GMO are possibly present in any biological sample (or material) including genetic material obtained (virus, fungi, bacteria, plant or animal cell, including the human). The biological sample can also be any culture medium wherein microorganisms, xenobiotics or pollutants are present, as well as such extract obtained from a plant or an animal (including a human) organ, tissue, cell or biological fluid (blood, serum, urine, etc).

The identification of specified GMO(s) in the sample may optionally be performed by quantification of event-specific genetic element in real-time PCR (Hernández et al., 2003, Transgenic Research 12: 179-189). Preferably the host is a plant as other organisms are not yet authorized as food.

According to the invention, the detection of the presence or absence of event-specific genetic element(s) and/or common genetic element(s), preferably further comprises the detection of plant elements which are specific of the plant species. Preferably, the plant elements (plant species) are selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice). The skilled person may also easily select other plant markers.

The identification of specified GMO(s) in the sample comprises a PCR amplification of the genetic elements and a detection of the amplified genetic elements on a micro-array. The micro-array contains 1, 2, 3, 4, 5, 6, 7, 8, 9, 10, 15, 20, 50, 100 or even more capture polynucleotide sequences, each capture polynucleotide sequence being able to recognize specifically an edge fragment of the specified GMO listed in table 1.

**Table 1 List A of the specified GMO.**

| | |
|---|---|
| The list comprises 18 GMOs including hybrids (designated by X). Equivalent names designating the same GMO are separated by the sign //. | |
| Bt176 | Mon531 |
| Mon810 | Mon15985 |
| Bt11 | Mon863 X Mon810 |
| Mon863 | Mon863 X Mon603 |
| GA21 | Mon603 X Mon810 |
| Mon603 // NK603 | GA21 X Mon810 |
| RRS // GTS 40/3/2 // Mon40-3-2 | Mon531 X Mon1445 |
| GT73 | Mon1445 |
| T45 | Mon15985 X Mon1445 |

Preferably, the capture polynucleotide sequence for the event-specific genetic element comprises at least 10 consecutive nucleotides having at least 5 nucleotides located on both sides of the event specific genetic element being an edge fragment.

The presence and absence of integrated exogenous nucleotide sequence (common and/or event-specific) forms the genetic map of a specified GMO, which is the organisation of the various genetic elements in the genome of the plant.

The invention links the presence and absence of the above-mentioned common and/or of the event-specific genetic elements detected in the assay(s) according to the invention, with a database having the genetic map of the specified GMOs. The identification of a specified GMO among other GMO present in the sample is performed by comparing the presence or absence of the different genetic elements with a database having the organisation of the genetic element (genetic map) of each specified GMO.

The genetic elements of the different genetic map(s) of the specified GMOs are arranged into a table and the result of the assay is compared one by one with the table data.

The invention preferably requires the table including the present genetic elements and the method for sorting out the identification of the organism from the experimental data.

The presence of unspecified GMO is deduced from the comparison for the presence and/or the absence of the common genetic elements measured in a given sample, with a genetic map and/or table according to the invention, whose substantiates all the common genetic elements of the specified GMOs.

An unspecified GMO is detected in a given sample when at least one detected common genetic element is not present in the genetic map recalling the presence of the common genetic elements of a specified GMO.

The common genetic elements are preferably selected from the group consisting of: P35S, T-Nos, Pnos-nptII, bla, nptII, Pat, Bar, Cry1Ab, Cry3Bb1 and EPSPS, this list being not limitative.

Preferably, the capture polynucleotide sequences for detecting the presence or absence of the said event-specific and common genetic element of the GMO contain a sequence specific of the genetic elements of at least 15, 16, 17, 18, 19, 20 or more nucleotides.

The capture polynucleotide sequence able to hybridise with its corresponding target nucleotide sequence is comprised between (about) 10 and (about) 49 nucleotides in length and is separated from the surface of the solid support by a spacer of at least 6.8 nm. The spacer is a sequence of between (about) 20 and (about) 150 nucleotides.

The binding between the target nucleotide sequence and capture polynucleotide sequence form a single spot signal at a specific location.

Preferably, the capture polynucleotide sequences are bound to the surface of the solid support by a direct covalent link according to a micro-array with a density of at least 4, preferably at least 10, 11, 12, 13, 14, 15, 16, 17, 18, 19, 20, 50, 80, 100, 150, 200, 250, 300, 400, 500, 1000, 4000, 10.000 or more, different capture nucleotide sequences/cm² insoluble solid support surface, said capture nucleotide sequences having advantageously a length comprised between (about) 10 to (about) 60 nucleotides, said sequence being specific for the target (which means that said bases of said sequence are able to form a binding with their complementary bases upon the sequence of the target by complementary hybridisation). Preferably, said hybridisation is obtained under stringent conditions, well-known to the person skilled in the art (Britten and Davidson (1990), Hybridization strategy. Nucleic acid hybridisation: A practical approach., B.D. Hames and S.J. Higgins. Washington DC, IRL Press: 3-14).

Advantageously, the target to be identified is labelled prior to its hybridisation with the single stranded capture polynucleotide sequences. Said labelling (with known techniques from the person skilled in the art) is preferably also obtained upon the amplified sequence previously to the denaturation (if the method includes an amplification step).

The labelled associated detections are numerous. A review of the different labelling molecules is given in W0 97/27317. They are obtained using either already labelled primer or by incorporation of labelled nucleotides during the copy or amplification step.

The most frequently used and preferred labels are fluorochromes like Cy3, Cy5 and Cy7 suitable for analysing a micro-array by using commercially available micro-array scanners (General Scanning, Genetic Microsystem,...). Radioactive labelling, cold labelling or indirect labelling with small molecules recognised thereafter by specific ligands (streptavidin or antibodies) are common methods. The resulting signal of target fixation on the micro-array is fluorescent, colorimetric, diffusion, electroluminescent, bio- or chemiluminescent, magnetic, electric like impedometric or voltametric (US-A-5,312,527).

A preferred method is based upon the use of the gold labelling of the bound target in order to obtain a precipitate or silver staining which is then easily detected and quantified by a scanner.

The density of the capture nucleotide sequence bound to the surface at a specific location is superior to 10 fmoles and preferably 100 fmoles per cm² of solid support surface.

According to the invention, the specified GMOs are selected from the group comprising: BT176, Mon810, Bt11, Mon863, GA21, Mon603, RRS, GT73, T45, Mon1445, Mon531, Mon15985, Mon863 X Mon810, Mon863 X Mon603, Mon603 X Mon810, GA21 X Mon810, Mon531 X Mon1445, Mon15985 X Mon1445, TC1507, Topas 19/2, H7-1, T25, DAS59122, TC1507 X Mon603, MS8 X RF3, MS1 X RF1, MS1 X RF2.

The specified GMOs are preferably all the GMOs accepted in the EU community.

The specified GMOs according to another aspect of the present invention are also those that will be specified in the future, as the skilled person will have no difficulties to implement databases, devices, design and synthesis of nucleotides and kit according to the invention.

Advantageously, the method also includes the appropriate means and steps to control a detection of possible presence of CaMV viruses (control of false positive detection) and possibly combined to an identification of the plant species.

The micro-array preferably comprise both capture polynucleotide sequences specific for the event-specific elements and polynucleotide sequences specific for common elements and/or specific for CaMV and / or specific for plant(s).

Advantageously, the detection of the presence or of the absence of the said event-specific and common genetic elements is performed at the same time.

Advantageously, the detection of the presence or of the absence of the said event-specific and common genetic elements and controls such as CaMV and/or plant-specific probes (such as Invertase (Maize), Lectin (Soybean), Acp1 (Cotton), UGPase (Potato), Cruciferin (Brassicaceae), Glutamine synthetase (Sugar beet) and Gos9 (Rice)) is performed at the same time.

The common genetic elements are amplified in total or in part. The event-specific sequences are amplified using specific primers, preferably one primer sequence being deduced from the host (plant) genome and the other primer sequence deduced from the inserted cassette.

Preferably, the amplification solution contains primer pairs both for specific and common sequence preferably 4 and still preferably 10 and even 20 or more primer pairs.

The amplification step used in the method according to the invention is advantageously obtained by well known amplification protocols, preferably selected from the group consisting of PCR, RT-PCR, LCR, CPT, NASBA, ICR or Avalanche DNA techniques. The preferred method for amplification of the genetic elements is PCR.

Preferably, the PCR comprises multiple specific primer pairs (multiplex PCR) which are able to amplify in the same PCR condition multiple genetic elements.

The genetic element is amplified by a specific primer pair, which is able to amplify a particular genetic element, preferably an event-specific genetic element and/or a common genetic element and/or a control element such as CaMV and / or plant-specific genes such as Invertase (Maize), Lectin (Soybean), Acp1 (Cotton), UGPase (Potato), Cruciferin (Brassicaceae), Glutamine synthetase (Sugar beet) and Gos9 (Rice).

The kit according to the invention is advantageously used for the identification of unspecified GMOs in a biological sample, said identification being obtained by detecting the genetic elements incorporated into the plants or animals or bacteria or yeast by genetic modification of their genome.

The kit of the invention is made of an insoluble solid support upon which single stranded capture polynucleotide sequences are bound, said capture polynucleotide sequences being specific of event-specific genetic elements and common genetic elements. The capture polynucleotide sequences are preferably immobilized on the solid support in the form of a micro-array having at least 10 and better 20 different capture (poly) nucleotide sequences. The event-specific genetic element is preferably an edge fragment and the common genetic elements are preferably selected from the group consisting of: P35s, T-nos, Pnos-nptII, bla, nptII, Pat, Bar, Cry1Ab, Cry3Bb1 and EPSPS.

The insoluble solid support of the kit comprises capture polynucleotide sequences for the detection of:
- at least 10 event-specific genetic elements of specified GMOs selected from the group consisting of : BT176, Mon810, Bt11, Mon863, GA21, Mon603, RRS, GT73, T45, Mon1445, Mon531, Mon15985, TC1507, Topas 19/2, H7-1, T25, DAS59122, MS8, RF3, MS1, RF1, RF2, and
- at least 3 common genetic elements selected from the group consisting of P35s, T-nos, Pnos-nptII, bla, nptII, Pat, Bar, Cry1Ab, Cry3Bb1 and EPSPS.

The capture polynucleotide sequences are preferably able to detect all the GMOs accepted in the EU community.

The insoluble solid support provided in the kit may also further comprise capture polynucleotide sequences for detection of plant elements which are specific of the plant species and/or of CaMV. Preferably, the plant elements (plant species) are selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice).

The insoluble solid support may be selected from the group consisting of glasses, electronic devices, silicon supports, plastic supports, compact discs, filters, gel layers, microbeads, metallic supports or a mixture thereof. Advantageously, said solid support is a glass slide which may comprise additional means (barcodes, markers, etc.) or media for improving the method according to the invention.

The solid support (biochip) can be inserted in a support connected to another chamber and automatic machine through the control of liquid solution based upon the use of microfluidic technology. By being inserted into such a microlaboratory system, it can be incubated, heated, washed and labelled by automates, even for previous steps (like extraction of DNA, amplification by PCR) or the following step (labelling and detection). All these steps can be performed upon the same solid support.

The single stranded capture polynucleotide sequences form a micro-array having a density of at least 4 single stranded capture polynucleotide sequences/cm² of solid support surface.

The kit further provides primer pairs for PCR amplification of the event-specific genetic element and the common genetic elements of the GMO to be detected as targets on the capture polynucleotide sequences immobilized on the solid support.

Preferably, the primer(s) and the specific portions of said GMO sequences to be amplified and detected are given in the examples 1 and 2. These primers amplified part or total sequence of at least 3 common genetic elements selected from the group consisting of: P35s, T-nos, Pnos-nptII junction, bla, nptII, pat, Bar, Cry1Ab, Cry3Bb1 and EPSPS, then detected on the micro-array.

According to a further aspect of the present invention, the method or kit (device) for identifying specified GMO selected from the group consisting of: BT176, Mon810, Bt11, Mon863, GA21, Mon603, RRS, GT73, T45, Mon1445, Mon531, Mon15985, Mon863 X Mon810, Mon863 X Mon603, Mon603 X Mon810, GA21 X Mon810, Mon531 X Mon1445, Mon15985 X Mon1445, TC1507, Topas 19/2, H7-1, T25, DAS59122, TC1507 X Mon603, MS8 X RF3, MS1 X RF1, MS1 X RF2.

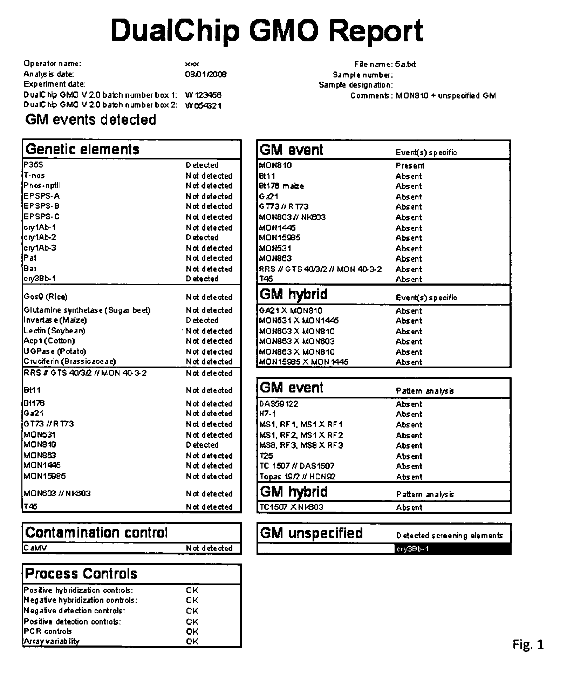

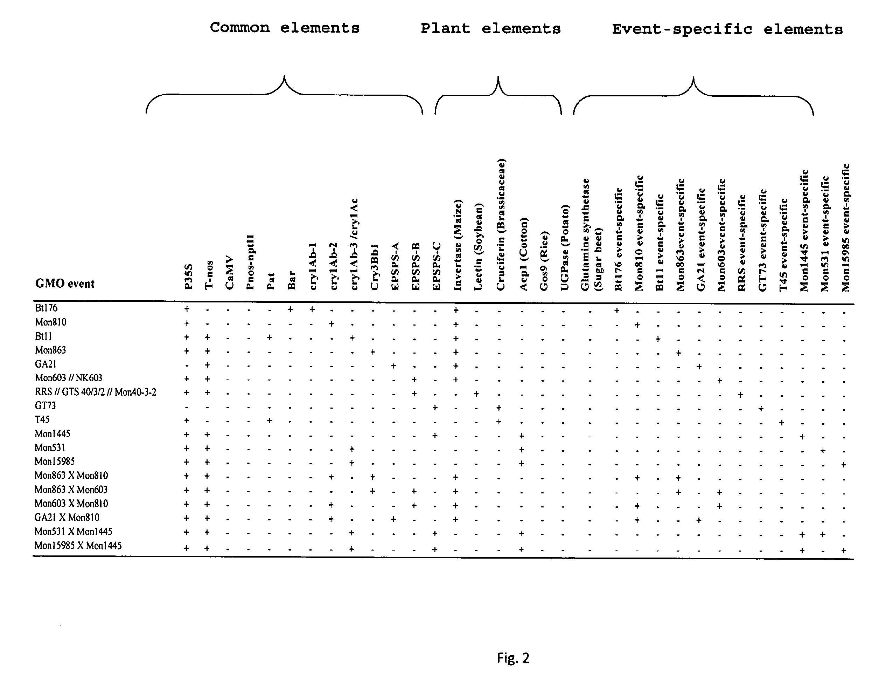

The detection of 27 specified GMOs according to the invention is presented in table 2 and in example 1. The micro-array for performing the detection and identification is presented in figure 1. The database for individual identification of the GMO is presented in figure 2. A list of GMO is provided in data base such are the BATS (http://www.gmo-watch.org/gmo-watch/GVOreport140703.pdf) and AgBios (http://www.agbios.com/dbaEe.php).

**Table 2 List B of the specified GMO as used in examples 1 and 2.**

| | |
|---|---|
| The list comprises 27 GMOs including hybrids (designated by X). Equivalent names designating the same GMO are separated by the sign //. | |
| Bt176 | GA21 X Mon810 |
| Mon810 | Mon531 X Mon1445 |
| Bt11 | Mon15985 X Mon1445 |
| Mon863 | TC1507 X Mon603 |
| GA21 | MS8 X RF3 |
| Mon603 // NK603 | Topas 19/2 // HCN92 |
| RRS // GTS 40/3/2 // Mon40-3-2 | H7-1 |
| GT73 | TC1507 // DAS1507 |
| T45 | T25 |
| Mon1445 | DAS59122 |
| Mon531 | Mon863 X Mon810 |
| Mon15985 | MS1 X RF1 |
| Mon603 X Mon810 | MS1 X RF2 |
| Mon863 X Mon603 | |

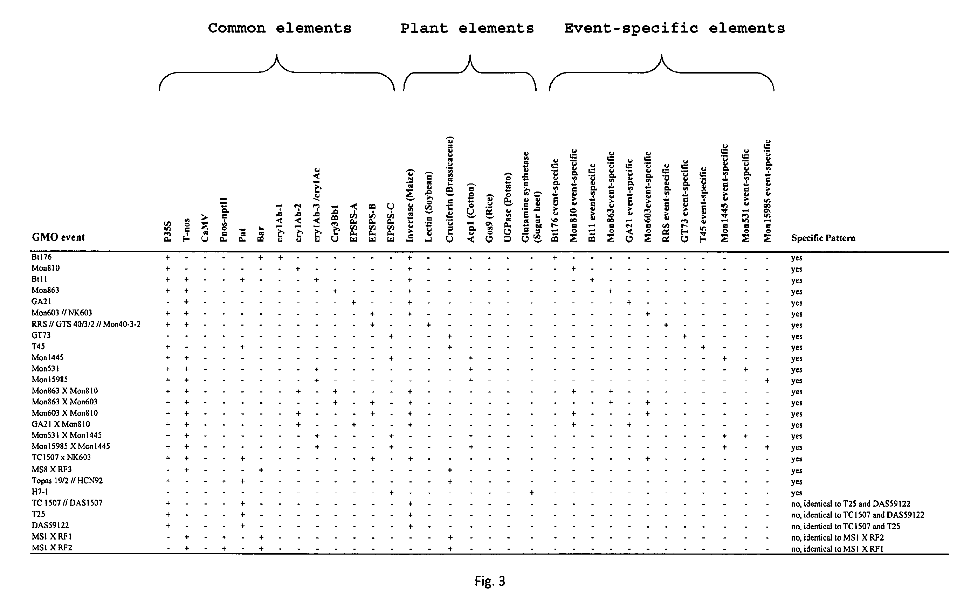

Genetics elements for the GMOs of Table 2 are shown in figure 3.

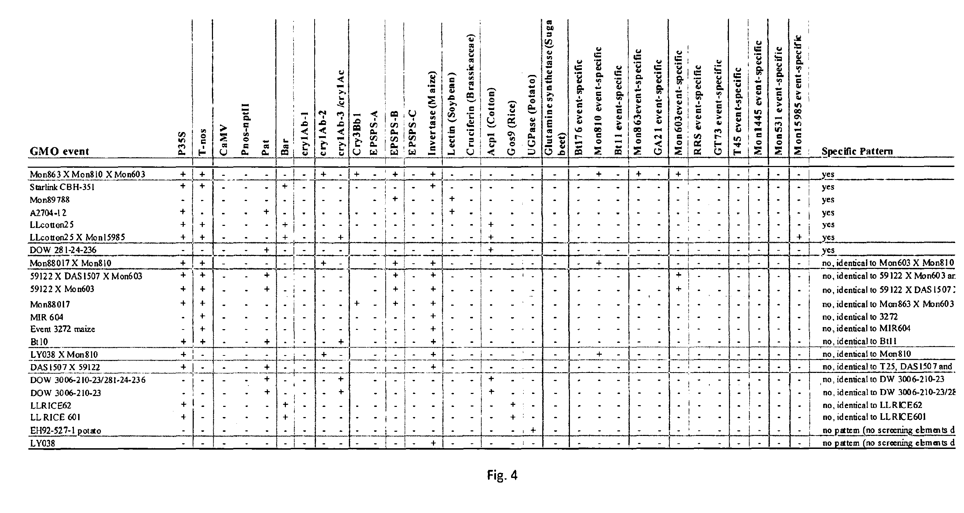

The Database also provides a list of the genetic elements present in the GMOs. The concept of specified and unspecified GMO is usually related to the accepted or non accepted GMO in the food, in the grain or in the animal feed. Specified GMOs are well known and the presence of genetic elements in their genetic map is known. Unaccepted and/or unknown GMO are a priori unspecified. The present invention allows detecting the presence of an unspecified GMO with or without prediction of its identity. Unspecified GMO are GMO outside the provided list. For some Unspecififed GMO, information is available in data base(s) so that indication is given by the presence of common genetic elements on their identity. For example, some unspecified GMO compared to list B are given in figure 4. Some of the unspecififed GMO have patterns which are specific of these GMO compared to all pattern of specified GMO of table 2 and of the other unspecified GMO of figure 4.

The quantification of the GMO is preferably performed by quantification of the signal for a genetic element present in the GMO. Preferably a comparison is done after said quantification of the amount of an event-specific element present in the sample compared to a plant genetic element. The ratio between the two allows a calculation of the percentage of GMO of one plant species in the sample.

Said quantification may also be done on common genetic elements.

Advantageously, the quantification of a specific organism is performed in term of copy number in the sample by comparing the amount of a target specific of the organism to a given number of standard copies added to the analyzed solution.

Advantageously the quantification of several common and/or event-specific elements is done in one sample and allows an improved detection in the case of the occurrence in one sample of several GMOs, being specified or not.

The quantification is performed by comparing the target and the standards, using tailed PCR primers (oligonucleotides) and then using second PCR primers (oligonucleotides) identical or complementary to the tail(s). The first tailed primers (oligonucleotides), having a common tail, bear a sequence complementary to the event-specific and/or common genetic elements and the standards or the plant marker as described by Knut et al. Nucleic Acids Res. 2003 Jun 1;31:e62). Amplification is first performed for 5 to 20 cycles with the tailed primers (oligonucleotides) and than for 5 to 40 cycles after destruction of the residual (unused / excess) tailed primer and addition of the said second PCR primer(s).

Alternatively, the amplification of the genetic element is performed by PCR using tailed primers and then using second primers identical or complementary to the tail(s). Advantageously, the tailed primers and the second tail primer(s) are both present from the beginning of the amplification with the tailed primers being at least in a concentration 5 times lower than the tail primer(s).

The quantification and the detection method for specified GMO are performed in the range of (about) 0.1 to (about) 5% of presence of GMO in the sample compared to non GMO plant. Typical standard curve of standardization comprises GMO sample ranging from (about) 0.1, 0.5, 1, 2 and (about) 5% GMO in non GMO species. Regulation for labelling and or detection of specified GMO containing sample is in the range of 0.9 and 0.5%. Unspecified GMO are GMO which are not recognized as accepted for the commercialization and can not be found in the sample. Detection methods are required to detect as low as 0.1 % of such unspecified GMO in the samples.

The kit also provides a computer program comprising program code means (possibly stored on a computer readable medium) for the determination of the possible presence of an unspecified GMO in a sample from the different signals obtained on the capture polynucleotide sequences, when said program is run on a computer. The computer program also comprises preferably a database with the composition of the different genetic elements of the specified GMOs.

Specified GMOs are first listed based on the presence of a particular genetic element in the assay corresponding to the GMO: the specified GMO for which an event-specific genetic element is detected in the assay and optionally, the specified GMO for which no event-specific genetic element is detected in the assay. The detection is then based on a specific pattern of common genetic elements for a given specified GMO.

Analysis steps for the determination of the presence of unspecified GMO in a sample performed on the experimental data is exemplified in example 1 and 2. The detection of the presence of an unspecified GMO in a sample is deduced from the list of detected common genetic element nucleotide sequences in the experiment and preferably comprised the following steps:
Step 1: GMO of group 1 (G1) detected in the sample based on their corresponding event-specific genetic element are listed.
   Step 1b: GMO of group 2 (G2) detected in the sample based on their corresponding pattern of common genetic element(s) are listed.
Step 2: The set of common genetic element(s) corresponding to the lists of GMO obtained in step 1 and 1b is determined.
Step 3: The list of the common genetic element(s) detected in the sample is provided from the experimental data.
Step 4: If at least one common genetic element detected in the sample in step 3 is not included in the set obtained in step 3, an unspecified GMO is detected. Step 1b is optional if the assay comprises event specific genetic element for all specified GMO.
The computer program comprising code means (preferably stored on a computer) preferably also lists unspecified GMO containing the common genetic element unaffected to specified GMO, by comparison of the data of step 4 with a data composition of unspecified GMOs. See figure 4 as an example of data composition or genetic map of unspecified GMO.

Algorithm in pseudo code:

n_spec_GMO <- number of specified GMO in the assay
n_common_seq <- number of common element(s) in the assay
n_detected_common_seq <- number of common element(s) in
the assay detected in the sample
// Split of GMO into 2 groups based on the presence of
specific element(s)
G1 <- empty
G2 <- empty
for i=1 to n_spec_GMO
if <GMOi has specific sequence>
G1 <- G1 + GMOi
else
G2 <- G2 + GMOi
endif

nG1 <- number of elements in G1
nG2 <- number of elements in G2

// Step 1
C <- empty // Let C be the set of G1 GMO detected in the
sample
for i = 1 to nG1
if <specific sequence of GMOi is detected in sample>
C <- C + GMOi

// Step 1b
D <- empty // Let D be the set of G2 GMO detected in the
sample
for i = 1 to nG2

    if <all common element(s) of GMOi pattern are detected
in sample>
            D <- D + GMOi

  Detected_GMO <- C + D
  N_Detected_GMO <- number of GMO in Detected_GMO
  F <- empty

  // Step 2
  for i = 1 to n_common_elements
    for j = 1 to nE
    if <Common_elementi is in the pattern of GMOj>
            F <- F + Common_seqi

  nF <- number of common element(s) in F
  G <- set of common element(s) in the assay detected in
the sample

  // Step 3
  For i = 1 to nF
    G <- G - Common_elementi

  //Step 4
  If <number of elements in G is > 0> then
    Unspecified GMO is present.

The present invention will be described in details in the following non-limiting examples in reference to the enclosed figures.

### Examples

### Example 1: Detection of specified GMOs on micro-array

### 1. The micro-array

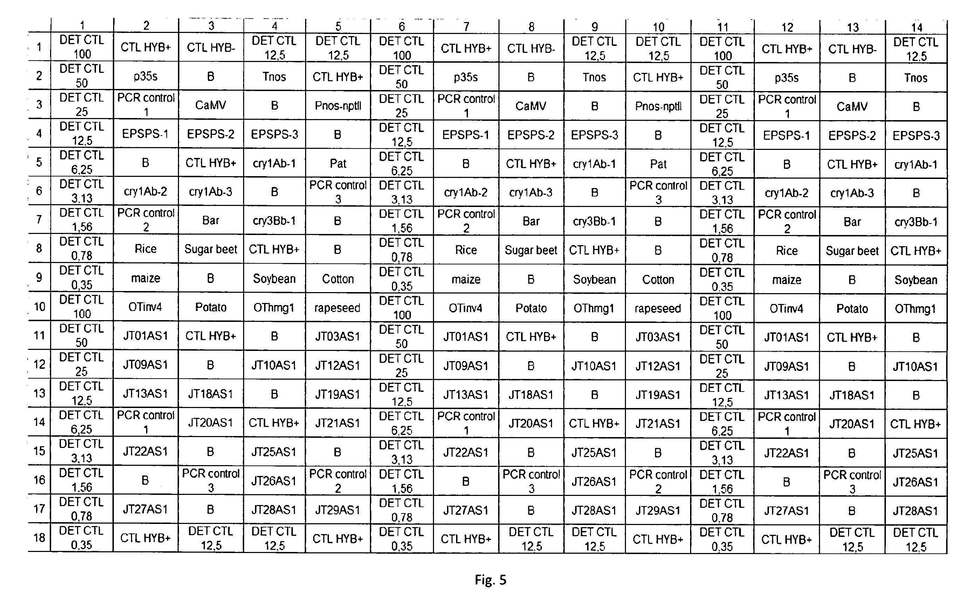

The design of the micro-array is presented in figure 5. It comprises common and event-specific genetic event nucleotide sequences. The micro-array was commercially available as DualChip GMO V 2.0 (Eppendorf AG, Hamburg, Germany). A glass slide comprised two identical micro-arrays. Each capture polynucleotide sequence (capture probe) was present in triplicate on the micro-array. The common genetic event sequences were described in the EP Patent application 05447115.6 incorporated herein by reference. These capture probes were able to identify genetically modified event nucleotide sequences by screening simultaneously multiple genetic elements: P35S, T-Nos, Pnos-nptII, pat, Bar, Cry1AB, Cry3Bb1, EPSPS, Invertase (Maize), Lectin (Soybean), Cruciferin (Rapeseed) and gut or hox (PCR control) sequences. The micro-array also includes advantageously a control element CaMV.

The micro-array is also able to detect a specific genetic event nucleotide sequence of a given specified GMO as provided in figure 3. The capture probe (polynucleotide sequence) Cry1Ab-1 is specific for BT176, the Cry1Ab-2 for Mon810 and the Cry1Ab-3/Cry1Ac for BT11, Mon531 and Mon15985. The capture probe (polynucleotide sequence) EPSPS-A is specific for GA1, the EPSPS-B for RRS and Mon603 and the EPSPS-C for GT73, Mon1445 and H7-1.

### 2 . Th e PCR

In order to amplify the sequences of the genetic elements that could be detected on the DualChip GMO V 2.0, 3 PCR reactions were processed according to the kit manual(Eppendorf AG, Hamburg, Germany). The primers used and PCR conditions were also described in EP Patent application 05447115.6.
PCR 1 (PCR S) allows the amplification of common genetic event nucleotide sequences of the GMO: 35S Promotor, Nos terminator, Pnos-nptII, CaMV, Pat, Bar, EPSPS, Cry1Ab, Cry3Bb1 and Gut (PCR S control) sequences.
PCR 2 (PCR P) allows the amplification of plant elements which are specific of the following plant species: Maize (Invertase gene), Soybean (Lectin gene), Brassicaceae (Cruciferin gene), Cotton (Acp1 gene), Rice (Gos9 gene), Sugar beet (Glutamine synhthetase (gs) gene), Potato (UGPase gene), gut (PCR P control).
PCR 3 (PCR E) allows the amplification of event-specific genetic element nucleotide sequences of 12 specified GMOs: GA21, Mon603, Bt11, RRS, Mon531, T45, GT73, Bt176, Mon810, MonN863, Mon15985, Mon1445 and hox (PCR E control).

The PCR amplification are performed in a final volume of 25 µl containing: 1X QIAGEN Multiplex PCR Master Mix containing HotStarTaq® DNA Polymerase, Multiplex PCR buffer and dNTP Mix, 0.15 µM of each biotinylated primer , 0.1 µM of each non-biotinylated primer (except for maize and sugar beet for which 0.3µM of biotinylated primer and 0.2 µM of non-biotinylated primer are used) and containing 200 ng of Genomic DNA that was extracted from a sample of Maize seeds using a CTAB-based method (Rogers, S.O. and Bendich, A.J. 1985. Extraction of DNA from milligram amounts of fresh, herbarium and mummified plant tissues. Plant Mol. Biol. 5: 69-76) and quantified using the "Quant-it^{™} PicoGreen dsDNA assay kit" (Invitrogen, USA) as described in the kit manual.

The DNA of the samples was denatured at 95 °C for 15 min. Then 32 cycles of amplification were performed consisting of 30 sec at 95 °C, 90 sec at 56 °C and 30 sec at 72 °C and a final extension step of 10 min at 72 °C.

### 3. Hybridization and colorimetric detection

In this experiment, the 3 PCR products (amplified sequences) were hybridized on two different micro-arrays of the same slide as replicates. The micro-arrays were covered with an hybridization frames and were treated as proposed by the manufacturer (Eppendorf AG, Hamburg, Germany). The DualChip GMO V 2.0 was hybridized with the 3 PCR products according to the to the kit manual (Eppendorf AG, Hamburg, Germany).
For each well, 9 µl of each PCR product (27 µl in total) was mixed in a 1.5 ml microtube with 5 µl of SensiHyb solution, 4 µl of hybridization control and 4 µl of water. 5 µl of 0.5 N NaOH was added and incubated for 5 min at room temperature. Then 50 µl of hybridization solution (Genomic HybriBuffer, Eppendorf, AG) was added and 100 µl of the hybridization mix solution was injected inside the hybridization frame. The frame was sealed and hybridization was performed for 1h at 60°C. The hybridization was immediately followed by the detection step. The colorimetric detection was performed using the Silverquant detection kit (Eppendorf, Hamburg, Germany).

The frames were removed and the slide was washed 2 times with Post Hybridization Buffer for 1 min and 3 times with Washing Buffer for 1 min. The slide was incubated for 10 min at temperature in Pre-Blocking Buffer and for 45 min at room temperature in the Diluted Gold-Conjugate. After incubation, the slide was washed 4 times with Washing Buffer for 1 min, then once with Rinsing Buffer for 1 min. Then, an equal volume of Silverquant A and B solutions were incubated for 5 min at room temperature. After incubation, the slide was washed 2 times with distilled water for 30 sec at room temperature and air dried. The slide was then inserted into the Silverquant scanner (Eppendorf, Hamburg, Germany) and scanning of the micro-array was performed according to the instruction manual. The Silverquant analysis software (Eppendorf, Hamburg, Germany) was used for processing the data of the micro-array.

A specified GMO for which event-specific capture probes (polynucleotide sequence) are available (figure 3) is considered as present in the sample when the corresponding event-specific capture probe is positive. For the specified GMO for which, no event-specific capture probes are available, the presence of the GMO is given by the correspondence between the presence of the common genetic event nucleotide sequences obtained on the micro-array and the genetic map of the GMO (figure 3).

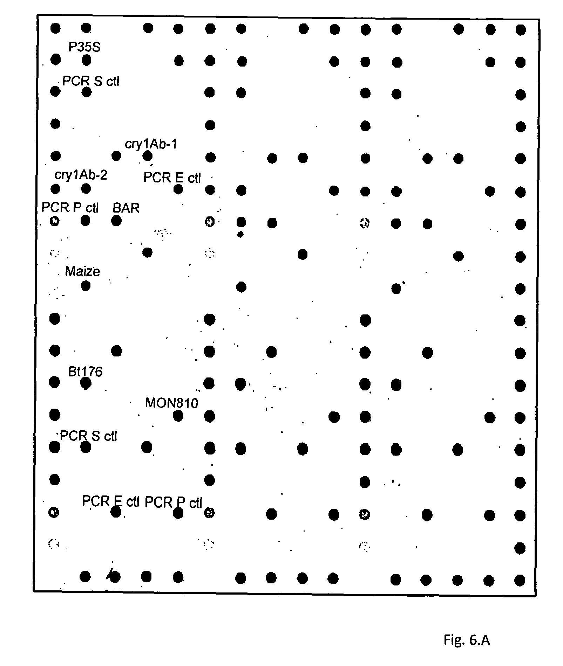

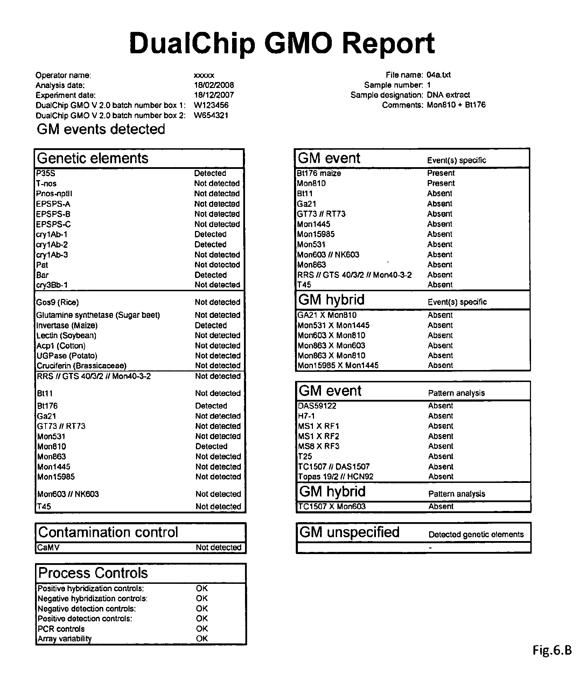

After hybridization and labelling with the Silverquant reagent, the micro-array is scanned with a 10 micrometer pixel resolution. The picture of such image is given in figure 6.A.

The quantification of the spots are then followed by a data analysis software for the treatment of the data, association of each spot with the gene, means of the triplicates, removal of the outliers, subtraction of background. At the end of the data analysis, a value is associated with each genetic element nucleotide sequence and its presence or absence is given by comparison of this experimental value with a fixed cut of which was settled as 2000 on a total scale of 65,000. The data are presented as a table as illustrated in figure 6.B (left column). They are then treated according to the steps proposed here above by another analysis software and the outcome of the data treatment is also given as a table as in figure 6.B (right column).

Two event-specific nucleotide sequences were detected, one specific of BT176 and the other one specific of Mon810. There was not particular pattern (of sequences) specific of other GMO so that the inventors concluded for the presence of BT176 and Mon810 in this sample. The analysis for the possible presence of unspecified GMO was negative since these two specified GMO account for all the common genetic element nucleotide sequences s detected in the assay, namely P35S, cry1Ab-1, cry1Ab-2 and Bar sequences.

The presence of Mon810 and BT176 sequences were confirmed by real time PCR assays performed individually for the different event-specific sequences. The results confirmed the presence of Mon810 at 0.4% and of BT176 at 6.5%.

### Example 2: Detection of unspecified GMOs on micro-array

The detection of the genetic element nucleotide sequences is performed with 3 PCR and capture probes as described in example 1 using the DualChip GMO V 2.0 (Eppendorf, Hamburg, Germany) and was performed on a sample of Maize seeds.

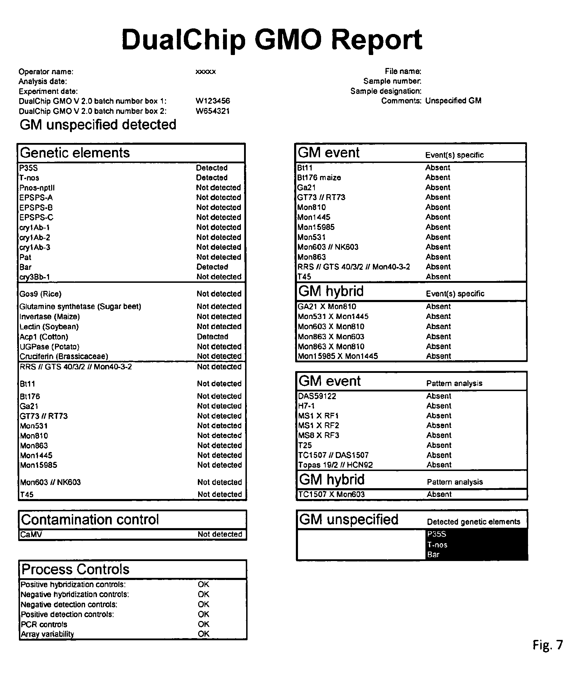

For the unspecified GMO, the analysis is done in the following way.
- First the presence of any specified GMO is detected as proposed here above based on the experimental results;
- Secondly, each of the common genetic element nucleotide sequences of the constructs is checked for its presence in the detected specified GMO;
- The presence of an unspecified GMO is detected when at least one common genetic element nucleotide sequence does not correspond to the information (pattern of nucleotide sequences) given by any of the specified GMO. The outcome of the data treatment is given in figure 7 (right column).
In this example, there is no specified GMO and the three common genetic element nucleotide sequences (P35S, T-Nos and Bar) are proposed as being associated with an unspecified GMO.

## Claims

1. A method for detecting the presence in a sample of an unspecified Genetically Modified Organism (GMO), said method comprising the steps of:
(1) possibly extracting from the sample genetics elements comprising at least a portion of an exogenous nucleotide sequence integrated into the genome of the GMO,
(2) detecting the presence or the absence in the sample of one or more common genetic element nucleotide sequence(s) comprising screening and/or gene specific sequences being part of the said portion integrated into the genome of the GMO, preferably selected from the group consisting of P35S, T-Nos, Pnos-nptII, bla, nptII, Pat, Bar, Cry1Ab, Cry3Bb1 and EPSPS nucleotide sequences,
(3) detecting the presence or absence in the sample of one or more event-specific genetic element nucleotide sequence(s),
(4) identifying the presence or the absence of all specified GMO(s) present in the sample from the detection step (2) and/or (3),
(5) associating each common genetic element detected in step (2) to the said specified GMO(s) present in the sample and identified in step (4),
(6) deducing the presence of an unspecified GMO in the sample, when at least one of the common genetic element detected in step (2) is not associated with any of the specified GMO identified in the sample.

2. The method of claim 1, which contains an event-specific element for at least 60%, preferably 80%, more preferably 100% for each of the specified GMO(s)

3. The method of claim 1 to 2, wherein the event-specific genetic element is an edge fragment.

4. The method of claim 1, wherein an unspecified GMO is detected in the sample when at least one detected common genetic element is not present in the genetic map recalling the presence of the common genetic elements of the specified GMO.

5. The method of claim 1, wherein the common genetic element nucleotide sequence(s) are selected from the group consisting of P35S, T-Nos, Pnos-nptII, bla, nptII, Pat, Bar, Cry1Ab, Cry3Bb1 and EPSPS nucleotide sequences.

6. The method according to any one of the claims 1 to 4, wherein the event-specific genetic element (nucleotide sequence) comprises at least 5 nucleotides located on both sides of the insertion point of an event-specific genetic element nucleotide sequence of the GMO.

7. The method according to any one of the claims 1 to 6, wherein the specified GMOs comprise at least 5, preferably at least 8 or all the specified GMOs selected from the group consisting of BT176, Mon810, Bt11, Mon863, GA21, Mon603, RRS, GT73, T45, Mon1445, Mon531, Mon15985, Mon863 X Mon810, Mon863 X Mon603, Mon603 X Mon810, GA21 X Mon810, Mon531 X Mon1445, Mon15985 X Mon1445, TC1507, Topas 19/2, H7-1, T25, DAS59122, TC1507 X Mon603, MS8 X RF3, MS1 X RF1, MS1 X RF2, preferably at least RRS, MON810, Bt11, MON531 and T25 or more preferably at least RRS, MON810, Bt11, MON531, MON15985, MON1445, T25 and Bt176.

8. The method of claim 1, wherein the step (2) and (3) are performed in the same assay.

9. The method according to any one of the claims 1 to 8, wherein the genetic elements are detected after amplification by PCR and preferably are quantified during the detection of the genetic elements nucleotide sequences present (step 2 and/or step 3) and GMO(s) abundance is deduced.

10. The method of claim 9, wherein the primer for the PCR amplification of the common genetic elements are consensus primers for the said amplification of elements being homologous in at least 2 different specified GMO(s).

11. The method according to any one of the claims 1 to 10, wherein the identification of the specified GMO(s) in the sample comprises a
- PCR amplification of the genetic elements nucleotide sequences, preferably using tailed primers and then using second primers identical or complementary to the said tail(s), and a
- detection of the amplified genetic elements on a micro-array.

12. The method according to any one of the claims 1 to 11, wherein the step(s) of detecting the presence or absence of one or more event-specific and/or common genetic element nucleotide sequence(s) is obtained by :
a) possibly extracting genetic elements corresponding to at least a portion of exogenous nucleotide sequence integrated into the genome of a GMO, from the sample;
b) amplifying or copying the said genetic element (or part(s) of it) nucleotide sequence(s) into target nucleotide sequences, having preferably a length comprised between about 50 and about 200 nucleotides;
c) possibly labelling the (possibly amplified) target nucleotide sequences;
d) putting into contact the (possibly labelled) target nucleotide sequences with capture polynucleotide sequences being specific of the amplified event specific genetic element(s) and being bound to an insoluble solid support, preferably a non porous solid support, containing at least 4 different bound capture polynucleotide sequences on different locations of solid support;
e) quantifying and/or recording for a target nucleotide sequence a signal at a specific location, said signal resulting from hybridization by complementary base pairing between the target nucleotide sequence and its corresponding capture polynucleotide sequence,
wherein the presence or the absence of a signal on a capture polynucleotide sequence at a specific location allows the detection and identification and/or quantification of the genetic element in the sample.

13. The method according of claim 12,
wherein the capture polynucleotide sequences able to hybridise with its corresponding target nucleotide sequence present a length comprised between about 10 and about 49 nucleotides and is separated from the surface of the solid support by a spacer of at least 6.8 nm, preferably a nucleotide sequence spacer of between about 20 and about 150 nucleotides.

14. The method according to claims 12 and 13, wherein the capture polynucleotide sequence for the event-specific genetic element comprises at least 10 consecutive nucleotides having at least 5 nucleotides located on both sides of the event specific genetic element being an edge fragment.

15. The method according to claim 11,
wherein the micro-array contains different capture polynucleotide sequences, each capture polynucleotide sequence being able to recognize specifically an edge fragment of the specified GMO listed in table 1.

16. The method according to any one of the claims 1 to 15, wherein the identification of the specified GMO(s) in the sample comprises a real time PCR amplification assay of the event-specific genetic elements.

17. The method according to any one of the claims 1 to 16, wherein the identification of the specified GMO(s) in the sample comprises:
a) amplifying or copying a common genetic element or part of it into target nucleotide sequences having a length comprised between about 50 and about 200 bases; by using primers which are able to amplify the said common genetic element being homologous with the common genetic element present in at least two different specified GMOs;
b) possibly labelling the obtained target nucleotide sequences;
c) putting into contact the obtained (and possibly labelled) target nucleotide sequences with single stranded capture polynucleotide sequences bound by a covalent link to an insoluble solid support at a specific location of a solid support surface;
d) detecting the binding of said (possibly labelled) target nucleotide sequences, by detecting a signal resulting from hybridization by complementary base pairing of said target nucleotide sequence and its corresponding capture polynucleotide sequence at the specific location, wherein the capture polynucleotide sequence(s) are bound to the insoluble solid support at a specific location according to a micro-array, having a density of at least 4 different bound single stranded capture nucleotide sequence(s)/cm² of solid support surface, wherein the capture polynucleotide sequence comprises a sequence having between 10 and 200 bases, which allows a specific hybridization with the target nucleotide sequence to be detected and/or quantified;
e) repeating the steps a) to d) for a second, third, fourth or more different common genetic elements specific of the specified GMO, and
f) identifying the specified GMO through the presence and absence of these different and multiple common genetic elements in the sample in comparison with their presence and absence in the GMO integrated exogenous nucleotide sequence.

18. The method according to any one of the claims 1 to 17, further comprising detection of organism element nucleotides sequences which are specific of the organism species, preferably selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice) nucleotide sequence.

19. A computer program for detecting the presence in a sample of an unspecified Genetically Modified Organism (GMO) and comprising program code means ( possibly stored on a computer readable medium) for performing the steps of:
1) listing specified GMO detected in the sample based on corresponding event-specific genetic element nucleotide sequence(s) and/or on corresponding pattern of common genetic element nucleotide sequence(s),
2) determining a set of common genetic elements corresponding to the specified GMO listed in step 1,
3) providing a list of common genetic element(s) detected in the sample,
4) comparing the list obtained in step 3 to the set obtained in step 2 and deducing a presence of an unspecified GMO if at least one common genetic element detected in the sample in step 3 is not included in the set obtained in step 2, when said program is run on a computer
The computer program of claim 23 further comprising the step of providing a list of unspecified GMO(s) containing the common genetic element unaffected to specified GMO by comparison of the data of step 4 with a data composition of unspecified GMOs.

20. The computer program of claim 19 in which the deducing step of the possible presence of an unspecified GMO of claim 21 is done comparing the different signals obtained on the capture polynucleotide sequences and a database collecting the genetic map of the different genetic element nucleotide sequence(s) of specified GMOs.

21. A diagnostic kit comprising
- a micro-array comprising an insoluble solid support upon which at least 10 different single stranded capture polynucleotide sequences are bound at a density of at least 4 single stranded capture polynucleotide sequences/cm² of solid support surface and
- primer pairs for (PCR) amplification of event-specific, common genetic element nucleotide sequences of the GMO and of organism element to be detected as targets on the capture polynucleotide sequences immobilized on the solid support surface,
- and means in the form of a computer program comprising program code means for performing the identification step of the method according to claim 19 and deducing step of the possible presence of an unspecified GMO in the sample when said program is run on a computer,
and, wherein the insoluble solid support (microarray) comprises capture polynucleotide sequences for the detection of:
- at least 10 event-specific genetic element nucleotide sequence(s) of specified GMOs selected from the group consisting of : BT176, Mon810, Bt11, Mon863, GA21, Mon603, RRS, GT73, T45, Mon1445, Mon531, Mon15985, TC1507, Topas 19/2, H7-1, T25, DAS59122, MS8, RF3, MS1, RF1, RF2 sequences , and
- at least 3 common genetic element nucleotide sequences selected from the group consisting of P35s, T-nos, Pnos-nptII, bla, nptII, Pat, Bar, Cry1Ab, Cry3Bb1 and EPSPS sequence
- at least 3 the plant species genetic element being the maize, soybean, cotton,patato, Brassicaceae, sugar beet and rice.

22. The kit according to claim 21, wherein the plant species genetic elements are selected from the group consisting of: Invertase (Maize), Lectin (Soybean), Acp1 (Cotton), UGPase (Potato), Cruciferin (Brassicaceae), Glutamine synthetase (Sugar beet) and Gos9 (Rice) nucleotide sequences.
